# EUROPÄISCHE PATENTANMELDUNG

(11) **EP 4 837 601 A1**
(43) Veröffentlichungstag der Anmeldung: **11.11.2026**
(21) Anmeldenummer: 25174297.9
(22) Anmeldetag: 05.05.2025
(51) Int. Cl.: A61M 5/142, A61M 5/168, A61M 5/36, A61M 39/28

(54) **INFUSIONSGERÄT MIT INTEGRIERTER GASBLASENERKENNUNG UND ZUGEHÖRIGES VERFAHREN**

(71) Anmelder: B. Braun Melsungen AG, 34212 Melsungen (DE)
(72) Erfinder: BRESSAU, Ernst, 34323 Malsfeld (DE); HOLLSTEIN, Rolf, 36211 Alheim-Heinebach (DE); KRAPF, Johannes, 36179 Bebra (DE)
(74) Vertreter: Tergau & Walkenhorst Intellectual Property GmbH

(57) **Zusammenfassung**

Die Erfindung betrifft ein Infusionsgerät (1) zur Förderung von Infusionslösungen, welches eine zuverlässige Erkennung und Klassifizierung von Gasblasen ermöglichen soll. Zu diesem Zweck umfasst das Infusionsgerät (1)
• eine Schlauchführung (6) für einen einlegbaren Infusionsschlauch (8),
• einen Gassensor (4) zur Detektion von Gasblasen in einem Flüssigkeitsstrom von Infusionsflüssigkeit im Infusionsschlauch (8),
• mindestens eine Applikationseinrichtung zur Erzeugung kurzzeitiger Druckschwankungen im Flüssigkeitsstrom, und
• eine Steuer- und Auswerteeinheit (14), die dazu eingerichtet ist, Sensorsignale des Gassensors (4) vor und/oder während und/oder nach der Erzeugung der Druckschwankungen auszuwerten, um anhand von charakteristischen Signalverläufen stehende Gasblasen zu identifizieren und diese von mit dem Flüssigkeitsstrom wandernden Gasmengen zu unterscheiden.

## Beschreibung

### Technisches Gebiet

Die Erfindung betrifft eine Vorrichtung und ein Verfahren zur Erkennung von Gasblasen, insbesondere Luftblasen, in einer Infusionsleitung eines Infusionssystems. In diesem Zusammenhang sind die genaue Dosierung sowie das Verhindern von größeren Lufteinschlüssen im Infusionsschlauch von besonderer Bedeutung, da Luftansammlungen im System zu gefährlichen Situationen für den Patienten führen können, wenn sie in dessen Blutbahn gelangen.

### Stand der Technik

Ein bekannter Stand der Technik umfasst Infusionsgeräte mit Luftsensoren, die den Infusionsschlauch überwachen und dabei Luftblasen detektieren, um eine Sicherheitsabschaltung oder einen Alarm auszulösen. In den gängigen Systemen werden die Luftblasen durch Ultraschall- oder optische Sensoren erkannt, wobei der Schlauch in der Regel horizontal im Bereich des Sensors verläuft. Diese Anordnung hat jedoch verschiedene Nachteile. Ein Hauptproblem besteht darin, dass sich bei bestimmten Schlauchführungen Luftblasen im Bereich des Sensors sammeln können und dort eine gewisse Größe erreichen, ohne weiter zu wandern (stehende Luftblasen). Dies führt unter Umständen zu einem Fehlalarm, bei dem die stehende Luftblase als deutlich größere Luftmenge interpretiert wird, obwohl tatsächlich keine gefährliche Luftansammlung vorliegt. Daher ist es notwendig, die stehenden Luftblasen von sich bewegenden Luftblasen zu unterscheiden.

### Aufgabe der Erfindung

Eine Aufgabe der Erfindung besteht darin, ein Infusionsgerät zur Verabreichung von Infusionslösungen zu schaffen, das die oben beschriebenen Nachteile des Standes der Technik überwindet. Insbesondere soll eine Lösung gefunden werden, bei der Luftblasen zuverlässig erkannt und bevorzugt auch analysiert und klassifiziert werden, so dass Fehlalarme oder eine Beeinträchtigung des Flüssigkeitsstroms vermieden werden.

### Lösung der Aufgabe

Die genannte Aufgabe wird erfindungsgemäß gelöst durch ein Infusionsgerät mit den Merkmalen des Anspruchs 1. Ein zugehöriges Verfahren ist Gegenstand von Anspruch 7.

Das Infusionsgerät umfasst eine Dosierpumpe, einen Gassensor, insbesondere einen Luftsensor, und eine Schlauchführung für einen einlegbaren Infusionsschlauch. Der Gassensor umfasst bevorzugt einen Ultraschallsender und einen Ultraschallempfänger. Diese sind derart angeordnet, dass Ultraschallwellen die Infusionsleitung innerhalb eines Messabschnitts durchqueren. Eine Steuer- und Auswerteeinheit wertet das empfangene Sensorsignal aus. Durch Erzeugung von kurzzeitigen Druckschwankungen im Infusionsschlauch können während der Förderung für einen kurzen Moment die stehenden Gasblasen, insbesondere Luftblasen, im Infusionsschlauch oder Strömungskanal bewegt werden. Dabei wird vor und/oder während und/oder nach, vorzugsweise permanent, das Sensorsignal aufgezeichnet und ausgewertet. Mit dem Wissen, wann die Druckschwankungen auftreten, ist das Identifizieren von stehenden Luftblasen durch das schwankende Sensorsignal möglich. Je nach Art und Menge der vorhandenen Luftblasen entstehen charakteristische Signalschwankungsprofile, die den jeweiligen Arten von Luftblasen, wie zum Beispiel stehende oder bewegliche Luftblase, zugeordnet werden können.

### Vorteilhafte Ausgestaltungen

Es wird darauf hingewiesen, dass sämtliche in Bezug auf die Vorrichtung beschriebenen Merkmale und Vorteile gleichermaßen für das beanspruchte Verfahren gelten, soweit sie technisch übertragbar sind. Insbesondere können die Merkmale der Steuer- und Auswerteeinheit, die Signalverarbeitung und die spezifischen Ausgestaltungen des Sensors in analoger Weise im Verfahren umgesetzt werden. Umgekehrt gelten alle im Rahmen des Verfahrens beschriebenen Schritte und ihre Vorteile auch für die Vorrichtung, da diese Schritte typischerweise durch entsprechende technische Merkmale der Vorrichtung realisiert werden.

Im Folgenden wird primär von Luftblasen gesprochen; es könnte sich aber auch um sonstige Gasblasen und Gasgemische handeln, die Blasen in einer umgebenden Flüssigkeit bilden.

Vorteilhafterweise werden die Druckschwankungen über die Dosierpumpe (kurzzeitig höheres Fördervolumen bzw. Volumenstrom) und/oder über eine motorisierte Schlauchklemme erzeugt. Letztere ist in der Lage, variablen Druck auf den Infusionsschlauch auszuüben. Durch diese Druckschwankungen werden während der Förderung für einen kurzen Moment die stehenden Luftblasen im Infusionsschlauch bewegt. Dabei lösen sich die Luftblasen, wandern weiter oder gehen wieder auf die Ursprungsposition zurück. In bevorzugter Ausführung werden dabei Druckschwankungen von einer Dauer von 1-10 ms appliziert.

Der Gassensor zur Detektion der Gasblasen umfasst in vorteilhafter Ausführung einen Ultraschallsensor, der wiederum einen Ultraschallsender und einen Ultraschallempfänger umfasst. Diese befinden sich auf gegenüber liegenden Seiten des Infusionsschlauchs und sind in vorteilhafter Ausführung in direktem Kontakt mit der äußeren Oberfläche des Infusionsschlauchs.

Die Auswertung findet bevorzugt automatisiert in der Steuer- und Auswerteeinheit statt. Alternativ können die Steuereinheit für den Ultraschallsender und die Auswerteeinheit für den Ultraschallempfänger auch separate Einheiten sein. Die Auswertung der Sensorsignale findet dann bevorzugt in der Auswerteeinheit statt. Die Steuer- und Auswerteeinheit ist in bevorzugter Ausführungsform zudem in der Lage, in Echtzeit bei Überschreiten eines zuvor festgelegten Grenzwerts bzw. vordefinierten Gasmenge und/oder bei einer erkannten spezifischen Gasblasenart ein Alarmsignal auszulösen. Dies erhöht die Patientensicherheit erheblich, da eine schnelle Reaktion möglich ist.

Alternativ oder zusätzlich kann die Steuer- und Auswerteeinheit bei der Auswertung der Sensorsignale zumindest teilweise auf einen Cloudservice oder dergleichen zurückgreifen. Bei Verwendung eines Universal-Computers oder programmierbaren Mikrocontrollers können die Auswertungsroutinen in Form eines Computerprogramms (Software) bereit-gestellt und bei Bedarf erneuert / ergänzt / verbessert werden (Updates).

Die erzeugten Druckschwankungen können in vorteilhafter Ausführungsweise durch weitere Methoden analysiert werden, wie z. B. durch Trendanalysen, Filtertechniken oder maschinelles Lernen. Das macht das System robust gegenüber Messfehlern oder Störungen. Es zeigt sich, dass insbesondere die kombinierte Anwendung von klassischen Verfahren und maschinellem Lernen eine außergewöhnlich hohe Sensitivität bei der Erkennung von Mikroblasen erzielt.

In bevorzugter Ausgestaltung umfasst die Steuer- und Auswerteeinheit ein selbstlernendes System, das historische Messdaten verwendet, um die Auswertelogik kontinuierlich zu optimieren. Dies hat den Vorteil, dass das System sich adaptiv an geänderte Bedingungen, wie Alterung der Sensoren oder abweichende Infusionsmedien, anpassen kann, ohne dass manuelle Neukalibrierungen erforderlich sind.

Zusammengefasst gewährleisten die mit der Erfindung erzielten Vorteile eine zuverlässige und differenzierte Erkennung von Luft- oder Gasblasen, die relativ einfach in bestehenden Systemen implementierbar ist. Die Echtzeitverarbeitung des Sensorsignals erlaubt das sofortige Auslösen von Alarmen bei detektierten makroskopischen Luftblasen. Die Erfindung stellt damit eine wichtige Verbesserung für die Sicherheit und Effizienz von Infusionstherapien dar.

### Kurzzusammenfassung der Figuren

Ein Ausführungsbeispiel der Erfindung wird nachfolgend unter Bezugnahme auf die beigefügten, rein schematischen Zeichnungen erläutert. Es zeigt:
- FIG. 1: ein Infusionsgerät gemäß der Erfindung in Frontalansicht, und
- FIG. 2: Beispiele für Luftsensorsignale, die bei verschiedenen Arten von Luftblasen nach Applikation von Druckpulsen entstehen.

Gleiche Elemente sind in allen Figuren mit denselben Bezugszeichen versehen.

### Detaillierte Beschreibung eines Ausführungsbeispiels

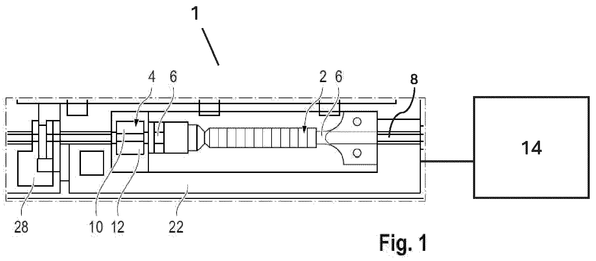

FIG. 1 zeigt eine Frontalansicht eines Infusionsgeräts 1, auch als Infusionspumpe bezeichnet, gemäß dem Stand der Technik bei geöffneter Frontklappe, die in der Darstellung nicht sichtbar ist. Das Infusionsgerät 1 umfasst eine Dosierpumpe 2, einen Gassensor 4 und eine Schlauchführung 6, die zum Einlegen eines Infusionsschlauchs 8 ausgelegt ist. Die Strömungsrichtung der Infusionslösung im eingelegten Infusionsschlauch 8 verläuft hier von rechts nach links.

Das Infusionsgerät 1 ist in seiner bestimmungsgemäßen Ausrichtung dargestellt, wobei das Gehäuse 22 horizontal ausgerichtet ist. Eine solche horizontale Anordnung erfolgt typischerweise auf einer ebenen Unterlage, beispielsweise einer Ablagefläche oder einem Medizingeräteträger. Alternativ kann das Gerät in ein Rackeinschubsystem integriert oder mittels einer Polklemme oder einer ähnlichen Fixiervorrichtung an einer vertikalen Halterung befestigt werden.

Die Dosierpumpe 2 ist hier im Beispiel als peristaltische Fördermechanik ausgeführt und wird von einer nicht dargestellten Antriebsmechanik betätigt. Es könnte sich aber auch eine andere Art von Fördereinrichtung handeln. Die Schlauchführung 6 erstreckt sich über die Breite des Gehäuses 22 und nimmt den Infusionsschlauch 8 so auf, dass dieser im Bereich der Dosierpumpe 2 und des Gassensors 4 horizontal verläuft. Der insbesondere als Luftsensor wirksame Gassensor 4 ist auf der linken Seite des Gehäuses 22 angeordnet und umfasst einen Ultraschallsender 10 und einem Ultraschallempfänger 12, die sich einander gegenüberliegen und im Betrieb den Infusionsschlauch 8 umgreifen.

Links vom Gassensor 4 ist eine motorisierte Schlauchklemme 28 angeordnet. Diese fungiert in bevorzugter Ausführung zusätzlich als Schlauchabsperr-Klemme. Zusätzlich kann die motorisierte Schlauchklemme 28 durch die Steuer- und Auswerteeinheit 14 angesteuert werden, wodurch durch Betätigen der Schlauchklemme 28 Druckschwankungen im Inneren des Infusionsschlauchs 8 erzeugt werden können.

Die Steuer- und Auswerteeinheit 14 ist bevorzugt in das Infusionsgerät 1 integriert, kann aber auch teilweise oder vollständig ausgelagert sein. Sie steuert bevorzugt auch die Dosierpumpe 2 an.

Insbesondere wenn der Infusionsschlauch 8 im Bereich des Luftsensors 4 horizontal verläuft, können sich darin entstehende Luftblasen sammeln und stagnieren. Dies kann zu einer unbeabsichtigten Ansammlung von Luft führen, die entweder einen unnötigen Alarm auslöst oder das Risiko birgt, dass tatsächlich vorhandene größere Luftblasen nicht rechtzeitig detektiert werden. Insbesondere können Luftblasen im Messbereich bzw. in der Messstrecke des Gassensors 4 stehen.

In der Infusionstechnik entstehen im Schlauchsystem und auch im Bereich der Gassensors kleinste Luftblasen u. a. durch Entgasung. Diese kleinen Luftblasen haften an der Schlauchwand und erzeugen im Bereich des Gassensors ein dauerhaftes "Luftsignal", welche unbegründete Alarme erzeugen.

Durch Applikation von kurzen Druckschwankungen durch die Dosierpumpe 2 oder die motorisierte Schlauchklemme 28 können die Luftblasen je nach Ausprägungsart mehr oder weniger stark in Bewegung versetzt werden, insbesondere solche, die sich direkt am Gassensor 4 befinden. Dabei entstehen je nach Art der Luftblase Änderungen im Ausgangssignal des Gassensors 4, die durch diesen detektiert werden können. Das Sensorsignal wird dabei vorzugsweise in kurzen Zeitintervallen wiederholt an die Steuer- und Auswerteeinheit 14 übermittelt und dort analysiert.

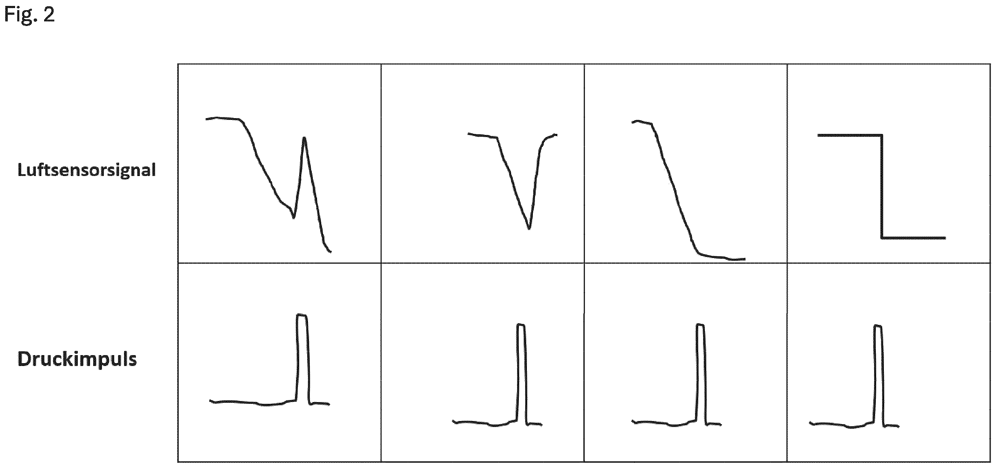

Die Verarbeitung und Auswertung des Sensorsignals findet bevorzugt in Echtzeit und automatisiert in der Steuer- und Auswerteeinheit 14 statt. In FIG. 2 sind die zeitlichen Verläufe exemplarischer Gassensorsignale bzw. Luftsensorsignale für verschiedene Arten von Luftblasen gezeigt, die sich im Sensorbereich befinden, während ein Druckimpuls appliziert wird. Der applizierte Druckpuls als Funktion der Zeit ist jeweils unten dargestellt. Die dazugehörigen zeitlichen Verläufe des Sensorsignals sind ebenfalls gezeigt, nämlich oben.

In einem ersten Beispiel in der linken Spalte ist eine stehende Luftblase am Sensor. Zu Beginn der Aufzeichnung liegen sowohl konstanter Druck als auch ein konstantes Luftsensorsignal vor. Nach gewisser Zeit beginnt sich eine Luftmenge im Sensorbereich anzusammeln, was durch eine Dämpfung des Luftsensorsignals erkennbar ist. Daraufhin wird ein Druckimpuls appliziert, erkennbar an einem kurzen, schlagartigen Ansteigen und nach kurzer Zeit wieder Abfallen des Drucksignals. Beim Applizieren eines Druckpulses verschiebt sich die Luftblase im vorliegenden Fall minimal, kehrt aber nach dem Druckpuls auf ihre Ausgangsposition zurück. Dieses Phänomen ist im Luftsensorsignal durch ein schnelles Ansteigen, gefolgt von einer schnellen Dämpfung des Luftsensorsignals erkennbar. Zusätzlich ist die Dämpfung nach dem Anstieg in diesem Fall stärker als der durch den Druckimpuls (und die damit verbundene Luftblasenverschiebung) erzeugte Signalanstieg, da die Luftblase in diesem Beispiel über die Zeit an Volumen gewinnt.

In der zweiten Spalte ist das Beispiel einer stehenden Luftblase am Sensor gezeigt, die nach der Applikation eines Druckpulses vom Flüssigkeitsstrom mitgerissen wird und somit vom Sensor entfernt wird. Dabei ist der Verlauf des Drucksignals zunächst analog zum ersten Beispiel. Auch ist zunächst das Sensorsignal konstant und eine Dämpfung tritt ein, sobald eine Luftblase im Sensorbereich entsteht und heranwächst. Im Unterschied zum ersten Beispiel resultiert der applizierte Druckimpuls in einer vollständigen Entfernung der Luftblase aus dem Sensorbereich, wodurch das Sensorsignal wieder auf den Wert zu Beginn der Aufzeichnung ansteigt.

In der dritten Spalte ist ein Beispiel gezeigt, in dem eine zuvor vorhandene Luftmenge im Sensorbereich beginnt, weiter anzuwachsen. Auch in diesem Beispiel ist der Verlauf des Drucks innerhalb des Infusionsschlauchs 8 analog zu den vorherigen Beispielen. Das Sensorsignal ist dabei zu Beginn konstant, da sich eine Luftmenge im Sensorbereich befindet, die an Volumen gewinnt. Zu einem gewissen Zeitpunkt nimmt das Volumen der Luftblase zunehmend zu und ein Druckimpuls wird appliziert. Aufgrund der großen Luftmenge hat die Druckschwankung hier keinen messbaren Einfluss auf das Luftsensorsignal und dessen Dämpfung schreitet voran, bis sich das Luftvolumen wieder stabilisiert und entsprechend das Luftsensorsignal einen größtenteils konstanten Wert einnimmt. Die Steuer- und Auswerteeinheit 14 interpretiert diesen Signalverlauf korrekt als große Luftmenge und löst daraufhin einen Luftalarm aus.

In der letzten Spalte ist eine plötzlich den Sensor passierende, große Luftmenge gezeigt. Der Verlauf des Drucks über die Zeit ist auch hier analog zu den vorherigen Beispielen. Das Luftsensorsignal ist hier konstant auf einem hohen Ausgangswert, entsprechend keiner messbaren Luftmenge im Sensorbereich. Über die Zeit wandert eine große Luftmenge durch den Infusionsschlauch, bis sie den Sensorbereich passiert. Daraufhin kommt es zu einem schlagartigen Abfall des Luftsensorsignals. Zu diesem Zeitpunkt wird ein Druckimpuls appliziert, welcher keinen messbaren Einfluss auf das Luftsensorsignal hat. Letzteres bleibt nach dem Eintreffen der Luftmenge auf einem konstant niedrigen Wert, was bedeutet, dass die Luftmenge immer noch im Sensorbereich ist. Die Steuer- und Auswerteeinheit 14 interpretiert das Luftsensorsignal als große Luftmenge, die durch den Infusionsschlauch 8 wandert und löst einen Luftalarm aus.

### Bezugszeichenliste

- 1.: Infusionsgerät
- 2.: Dosierpumpe
- 4: Gassensor
- 6: Schlauchführung
- 8: Infusionsschlauch
- 10: Ultraschallsender
- 12: Ultraschallempfänger
- 14: Steuer- und Auswerteeinheit
- 22: Gehäuse
- 28: Schlauchklemme

## Patentansprüche

1. Infusionsgerät (1) zur Förderung von Infusionslösungen, umfassend:
• eine Schlauchführung (6) für einen einlegbaren Infusionsschlauch (8),
• einen Gassensor (4) zur Detektion von Gasblasen in einem Flüssigkeitsstrom von Infusionsflüssigkeit im Infusionsschlauch (8),
• mindestens eine Applikationseinrichtung zur Erzeugung kurzzeitiger Druckschwankungen im Flüssigkeitsstrom, und
• eine Steuer- und Auswerteeinheit (14), die dazu eingerichtet ist, Sensorsignale des Gassensors (4) vor und/oder während und/oder nach der Erzeugung der Druckschwankungen auszuwerten, um anhand von charakteristischen Signalverläufen stehende Gasblasen zu identifizieren und diese von mit dem Flüssigkeitsstrom wandernden Gasmengen zu unterscheiden.

2. Infusionsgerät (1) gemäß Anspruch 1, wobei die Applikationseinrichtung eine motorisierte Schlauchklemme (28) umfasst, welche variablen Druck auf den Infusionsschlauch (8) ausüben kann und durch die der Infusionsschlauch (8) geführt wird.

3. Infusionsgerät (1) gemäß einem der vorherigen Ansprüche, wobei die Applikationseinrichtung eine Dosierpumpe (2) umfasst, welche variablen Druck auf den Infusionsschlauch (8) ausüben kann.

4. Infusionsgerät (1) gemäß einem der vorherigen Ansprüche, wobei die Dauer der Druckschwankungen 1-10 ms beträgt.

5. Infusionsgerät (1) gemäß einem der vorhergehenden Ansprüche, wobei der Gassensor (4) einen Ultraschallsender (10) und einen Ultraschallempfänger (12) umfasst.

6. Infusionsgerät (1) gemäß einem der vorhergehenden Ansprüche, welches bei einer vordefinierten detektierten Gasmenge ein Alarmsignal auslöst.

7. Verfahren zur Erkennung und Unterscheidung von Gasblasen in einem Flüssigkeitsstrom von Infusionsflüssigkeit, umfassend die Schritte:
• Erzeugen eines Flüssigkeitsstroms von Infusionsflüssigkeit durch einen Infusionsschlauch (8) mittels einer Fördereinrichtung,
• Detektion von Gasblasen im Flüssigkeitsstrom durch einen Gassensor (4),
• Erzeugen kurzzeitiger Druckschwankungen im Flüssigkeitsstrom,
• Erfassen eines Sensorsignals des Gassensors (4) vor und/oder während und/oder nach der Erzeugung der Druckschwankungen,
• Identifizieren stehender Gasblasen und Unterscheiden von wandernden Gasmengen basierend auf charakteristischen Signalverläufen des Sensorsignals.

8. Verfahren gemäß Anspruch 7, wobei die Druckschwankungen durch eine motorisierte Schlauchklemme (28) erzeugt werden.

9. Verfahren gemäß Anspruch 7, wobei die Druckschwankungen durch Variation der Flussrate im Infusionsschlauch (8) erzeugt werden.

10. Verfahren gemäß einem der Ansprüche 7 bis 9, wobei die Dauer der Druckschwankungen 1-10ms beträgt.

11. Verfahren gemäß einem der Ansprüche 7 bis10, wobei bei Überschreitung eines zuvor festgelegten Schwellenwerts des detektierten Gasvolumens ein Alarmsignal ausgelöst wird.
